# FASCICULE DE BREVET EUROPEEN

(11) **EP 1 035 829 B1**
(45) Date de publication et mention de la délivrance du brevet: **15.05.2002**
(21) Numéro de dépôt: 98958300.0
(22) Date de dépôt: 02.12.1998
(51) Int. Cl.: A61K 7/48, A61K 7/06

(54) **UTILISATION DE GOMME DE DIPHENYLDIMETHICONE DISSOUTE DANS UNE SILICONE NON VOLATILE DE TYPE PHENYLTRIMETHICONE POUR LA FABRICATION D'UNE COMPOSITION COSMETIQUE OU PHARMACEUTIQUE, NOTAMMENT DERMATOLOGIQUE COMPRENANT UNE PHASE GRASSE**
VERWENDUNG EINES DIPHENYLDIMETHICONGUMMIS GELÖSST IN EINEM NICHTFLÜCHTIGEN SILIKON DES PHENYLTRIMETHICONTYPS ZUR HERSTELLUNG VON EINEM KOSMETISCHEN ODER PHARMAZEUTISCHEN , INSBESONDERE DERMATOLOGISCHEN ZUSAMMENSETZUNG DIE EINE FETTPHASE ENTHÄLT
USE OF A DIPHENYLDIMETHICONE DISSOLVED IN A NON-VOLATILE SILICONE SUCH AS PHENYLTRIMETHICONE FOR MAKING A COSMETIC OR PHARMACEUTICAL, IN PARTICULAR DERMATOLOGICAL, COMPOSITION COMPRISING A FATTY PHASE

(30) Priorité: 02.12.1997 FR 9715177
(43) Date de publication de la demande: 20.09.2000
(73) Titulaire: PARFUMS CHRISTIAN DIOR, 75008 Paris (FR)
(72) Inventeur: WILLEMIN, Claudie, F-75008 Paris (FR); BURTIN, Frédéric, F-45000 Orléans (FR)
(74) Mandataire: Portal, Gérard
(86) Numéro de dépôt international: FR9802591
(87) Numéro de publication internationale: WO9927903

(56) Documents cités:
- EP-A- 0 756 864
- EP-A- 0 756 865

## Description

La présente invention concerne essentiellement l'utilisation de gomme de diphényldiméthicone dissoute dans une silicone non volatile de type phényltriméthicone pour la fabrication d'une composition cosmétique ou pharmaceutique, notamment dermatologique, comprenant une phase grasse.

Dans l'état de la technique antérieure, on connaît par le document WO 97/12584 des compositions de silicone ainsi que leur méthode de préparation et leur emploi en cosmétique pour traiter la peau ou les cheveux.

Les documents EP-A-0 756 865 et EP-A-0 656 864 divulguent des formulations cosmétiques dont la phase grasse comprend une huile de silicone phénylée (qui peut être un polyphénylméthylsiloxane ou un phényltriméthicone, ou un mélange de différentes huiles siliconées phénylées) solubilisée dans une huile volatile (qui peut être choisie parmi les huiles volatiles hydrocarbonées ou siliconées). Ces compositions peuvent en outre comprendre d'autres corps gras siliconés comme des gommes de silicone ou silicones modifiées par des groupements aromatiques.

Dans le cadre de l'art antérieur, ces gommes de silicone sont solubilisées dans une huile de silicone volatile et sont, par exemple, disponibles sur le marché sous la dénomination commerciale MIRASIL® C-DPDM, de RHONE-POULENC, France, c'est-à-dire une diphényldiméthicone, solubilisée dans du cyclométhicone.

Les gommes de silicone sont particulièrement intéressantes en cosmétique, car elles apportent des qualités, qui sont essentiellement recherchées en cosmétique comme l'onctuosité, un pouvoir glissant, une brillance, un caractère filmogène, un effet de résistance à l'eau, un effet hydrofuge, un effet d'adhérence, un effet conditionneur, un effet anti-statique, un effet adoucissant conférant un toucher doux, et un caractère substantif, et une bonne tenue dans le temps.

L'invention a pour but principal de fournir une solution qui permette de faciliter l'incorporation de gomme de silicone dans des compositions cosmétiques ou même pharmaceutiques, notamment dermatologiques, comprenant une phase grasse.

L'invention a encore pour but principal de fournir une solution qui permette de réaliser l'incorporation aisée d'une gomme de silicone dans des compositions cosmétiques ou pharmaceutiques, notamment dermatologiques, en augmentant les possibilités de formulation de ces produits avec un maintien ou de préférence une amélioration de la compatibilité avec d'autres ingrédients de la phase grasse et en particulier les corps gras tels que les huiles par les cires, en obtenant ainsi des produits de très bonne qualité.

La présente invention fournit pour la première fois une solution à l'ensemble des problèmes techniques énoncés ci-dessus d'une manière particulièrement simple, peu coûteuse, utilisable à l'échelle industrielle cosmétique ou pharmaceutique, notamment dermatologique.

Ainsi, selon un premier aspect, la présente invention concerne l'utilisation d'une gomme silicone de type diphényldiméthicone solubilisée dans une huile de silicone non volatile de type phényltriméthicone pour la fabrication d'une composition cosmétique ou pharmaceutique, notamment dermatologique, comprenant une phase grasse.

Dans le cadre de l'invention, la gomme de silicone de type diphényldiméthicone présente la formule (I) suivante : 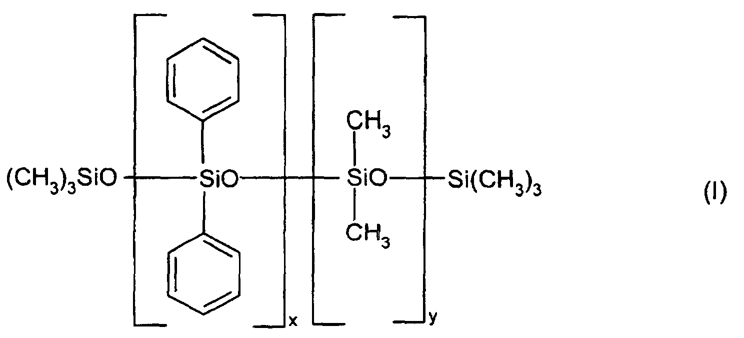 dans laquelle x représente le nombre de motifs de récurrence qui est généralement peu élevé, de préférence inférieur à environ 500, avantageusement compris entre environ 50 et environ 150, encore mieux compris entre environ 80 et environ 120, tandis que y représente le nombre de motifs de récurrence qui est généralement plus élevé, de préférence au moins égal à 1 000 et encore mieux compris entre environ 1 000 et environ 2 000.

Par ailleurs, dans le cadre de l'invention, l'huile silicone solubilisante non volatile de la gomme de silicone est une phényltriméthicone de formule (II) ci-après : 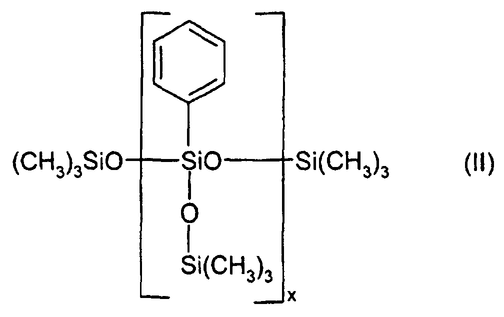 dans laquelle x représente le nombre de motifs de récurrence qui permet d'obtenir une viscosité comprise entre environ 0,1 m²/s et environ 0,4 m²/s (entre environ 10 et environ 40 centiStokes) à une température de 25°C. De préférence, x aura une valeur moyenne comprise entre 2 et 3.

Dans le cadre de l'invention, la proportion relative entre la gomme de silicone diphényldiméthycone et l'huile de silicone solubilisante phényltriméthicone peut varier dans de larges limites. Cependant, cette proportion relative sera avantageusement comprise entre 0,1 à environ 20 % en poids de gomme de silicone, par rapport au mélange de gomme de silicone et d'huile de silicone solubilisante phényltriméthicone, et encore mieux entre environ 5 et environ 15 en poids et idéalement environ 15 % en poids.

Cependant, selon d'autres variantes de réalisation de l'invention, le mélange binaire de gomme de silicone de type diméthicone et d'huile de silicone solubilisante de type phényltriméthicone ne constituera qu'une partie de la phase grasse, avantageusement de 0,1 à 100 %, mieux de 1 % à 75 %, encore mieux de 5 à 50 % de la phase grasse.

Ainsi, dans le cadre de l'invention, la phase grasse peut être entièrement constituée du mélange binaire de la gomme de silicone précitée solubilisée dans l'huile de silicone de type phényltriméthicone.

Cette phase grasse comprendra avantageusement au moins une autre huile silicone ou non silicone et éventuellement un corps gras qui peut être avantageusement d'origine végétale.

On peut utiliser comme huile non silicone plus généralement un corps gras non silicone, au moins un ester de glycérol et d'acide gras sous forme de mono-, di- ou tri-glycéride ; un ester gras d'acide et d'alcool, l'acide et/ou l'alcool étant gras.

Dans le cadre de la présente invention, c'est-à-dire de la description et des revendications, on entend par « acide gras » un acide ayant généralement de 5 à 30 atomes de carbone, à chaîne linéaire ou ramifiée ou encore cyclique, saturée ou insaturée, pouvant comporter un ou plusieurs noyaux aromatiques, en particulier un ou plusieurs groupes phényles ou benzyles.

D'autre part, on entend également dans le cadre de l'invention, c'est-à-dire de la description et des revendications, par l'expression « alcool gras », un alcool ayant au moins 8 atomes de carbone, de préférence entre 8 et 30 atomes de carbone.

Dans le cadre de l'invention, on peut également utiliser l'acide gras seul ayant dans ce cas un nombre d'atomes de carbone plus élevé, en particulier d'au moins 12 atomes de carbone, tel que précédemment défini, et dans le cadre de l'emploi d'un alcool gras seul, également ayant dans ce cas au moins 12 atomes de carbone, tel que précédemment défini.

On peut également incorporer dans les compositions cosmétiques ou pharmaceutiques, notamment dermatologiques de l'invention, des agents tensioactifs ou émulsionnants de type ionique ou non ionique, tels que des esters de sorbitan, des alcools gras polyoxyéthylénés, des esters de sucrose.

Selon une variante de réalisation avantageuse de l'invention, les compositions cosmétiques ou pharmaceutiques, notamment dermatologiques, peuvent également contenir au moins un additif cosmétiquement ou pharmaceutiquement, notamment dermatologiquement acceptable tel qu'un agent épaississant, un ester d'acide gras en particulier des esters d'acide gras et de glycérol, d'autres huiles silicones volatiles ou non volatiles, des filtres solaires, des huiles végétales, des huiles synthétiques, des parfums, des agents conservateurs.

Les compositions selon l'invention peuvent être formulées sous diverses formes bien connues à l'homme de l'art tel que gel, lait, crème, lotion de consistance variable adaptée aux utilisations envisagées.

Dans le cadre de l'invention, les compositions cosmétiques ou pharmaceutiques, notamment dermatologiques seront plus particulièrement utilisées dans le domaine du soin de la peau, en particulier du visage ou du corps.

Selon une autre variante, les compositions cosmétiques ou pharmaceutiques, notamment dermatologiques selon l'invention, peuvent être utilisées pour le soin des cheveux.

Selon un deuxième aspect, la présente invention couvre aussi des compositions cosmétiques, pharmaceutiques, notamment dermatologiques, caractérisées en ce qu'elles comprennent une phase grasse comprenant une gomme de silicone de type diphényldiméthicone solubilisée dans une huile de silicone non volatile de type phényltriméthicone, en particulier tel que précédemment défini.

Selon un mode de réalisation avantageux de l'invention, cette composition est une composition pour le soin de la peau et comprend en outre dans la phase grasse un composé choisi parmi le groupe consistant de :
- un ester de glycérol, avantageusement un triglycéride liquide à la température ambiante, telle que le tricaprilate/caprate de glycérol, un triglycéride d'acide caprilique/caprique/succinique, en particulier à une concentration d'environ 1 à environ 10 % en poids,
- une huile de silicone de type phényltriméthicone ou diméthicone en particulier à une concentration comprise entre environ 1 et environ 10 % en poids, mieux de l'ordre de 2 à 5 % en poids,
- une huile végétale, avantageusement une huile de jojoba ou une huile de graine "d'herbe de la prairie" (en anglais "meadowfoam seed oil"), en particulier à une concentration de 0,5 à 10% en poids, mieux d'environ 1 à environ 5 % en poids,
- au moins un agent gélifiant, avantageusement un polysaccharide tel qu'une gomme de xanthane ou un polymère acrylique de type carbomer, ou un mélange gélifiant à base d'isoparaffine, de polyacrylamide et d'alcool laurique polyoxyéthyléné, en particulier disponible dans le commerce sous la dénomination commerciale de SEPIGEL 305 commercialisé par la société SEPPIC, en particulier à une concentration comprise entre environ 0,1 et environ 10 % en poids, mieux entre environ 0,1 et environ 5 % en poids,
- un agent émulsionnant/tensioactif de type non ionique tel que stéarate de sorbitan ou un stéarate de sorbitan polyoxyéthyléné ou un ester de sucrose tel que le stéarate de sucrose, en particulier à une concentration comprise entre environ 0,5 et environ 5 % en poids;

Selon une autre variante de réalisation de l'invention, dans le cadre d'une composition destinée à la protection solaire, celle-ci comprend en outre les ingrédients suivants :
- au moins un filtre solaire chimique tel que le méthoxycinnamate d'octyle, le butylméthoxydibenzoylméthane, la benzophénone-3 et la benzophénone-4.

Dans le cas de l'emploi du méthoxycinnamate d'octyle on utilisera en particulier une concentration comprise entre 0,1 et 10 % en poids, dans le cas du butylméthoxydibenzoylméthane on utilisera une concentration avantageusement comprise entre 0,1 et 5 % en poids, en particulier de l'ordre de 2 à 3 % en poids, pour la benzophénone-3 ou -4 en particulier une concentration comprise entre 0,1 et 5 % en poids,
- au moins un filtre solaire physique tel que l'oxyde de titane micronisé en particulier à une concentration comprise entre 0,1 et 20 % en poids ; l'oxyde de zinc micronisé en particulier à une concentration comprise entre 0,1 et 20 % en poids, ou toutes particules hybrides réalisées à partir d'un mélange d'oxyde de titane ou de zinc avec un autre composant en particulier lorsque ces oxydes sont fixés à la surface d'une particule polymérique, par exemple des particules de polyéthylène ou de polypropylène comportant à leur surface de tels oxydes.

De telles compositions cosmétiques, pharmaceutiques, notamment dermatologiques, peuvent être par exemple constituées par des compositions capillaires, sous quelques formes que ce soient, par exemple gel ou crème, des compositions destinées à être appliquées sur la peau, topiquement, sous la forme d'émulsion de type eau-dans-huile ou huile-dans-eau, sous la forme d'un gel, en particulier de gel transparent, ou encore de lotion, ou encore des formes solides anhydres telles que bâtonnets ou sticks, ou encore des produits pour les ongles.

Selon un deuxième aspect, la présente invention concerne aussi des compositions cosmétiques ou pharmaceutiques, notamment dermatologiques, comprenant une phase grasse comprenant elle-même un mélange de gomme de silicone de type diphényldiméthicone solubilisée dans une huile de silicone de type phényltriméthicone, plus particulièrement telle que précédemment définie.

Naturellement, ces compositions cosmétiques ou pharmaceutiques, notamment dermatologiques, peuvent contenir d'autres ingrédients actifs que l'homme de l'art souhaitera incorporer ainsi que divers excipients ou véhicules cosmétiquement ou pharmaceutiquement, dermatologiquement, acceptables, qui sont biens connus de l'homme de l'art.

D'autres buts, caractéristiques et avantages de l'invention apparaîtront également clairement à partir de la description suivante faite en relation avec divers exemples de réalisation actuellement préféré de l'invention, donnés seulement à titre d'illustration et qui ne sauraient donc en aucune façon limiter la portée de l'invention.

Dans les exemples, les proportions sont données en poids, la température est la température ambiante et la pression est la pression atmosphérique, sauf indication contraire.

### Exemple 1 de l'invention

### Composition cosmétique selon l'invention de maquillage type rouge à lèvres

Cette composition est formulée à partir des ingrédients suivants :
- composition binaire de gomme de silicone diphényldiméthicone solubilisée dans une huile de silicone phényltriméthicone, la gomme de silicone représentant 15 % du mélange 5 %
- isostéarate d'isostéaryle 20 %
- palmitate d'octyle 10 %
- cire microcristalline 8 %
- cire de candelilla 5 %
- cire d'abeille 5 %
- tricaprylate/caprate de glycérol 4 %
- octyl méthoxy cinnamate 3 %
- ricinoléate de cétyle 3 %
- oxydes de fer 5 %
- pigments organiques sur laques 1,5 %
- nacres 6 %
- parfum 0,3 %
- huile de ricin avec conservateur qsp 100 %

Cette composition cosmétique est préparée de la manière suivante :

On incorpore tous les composants gras y compris la composition binaire de l'invention, à l'exception des pigments et nacres, que l'on mélange tout en chauffant jusqu'à une température de 85-90°C afin de faire fondre les substances solides à température ambiante, telles que les cires, jusqu'à obtention d'une homogénéité complète.

On rajoute ensuite les pigments et nacres préalablement dispersés dans une partie d'huile de ricin jusqu'à obtention d'une homogénéité parfaite.

On coule ensuite à chaud dans des moules permettant de donner la forme finale des rouges à lèvres et on laisse refroidir avant de démouler, de manière bien connue à l'homme de l'art.

### Exemple 2 selon l'invention

### Composition cosmétique selon l'invention de maquillage type rouge à lèvres

Cette composition est formulée à partir des ingrédients suivants :
- composition binaire de l'exemple 1 10 %
- isostéarate d'isostéaryle 20 %
- palmitate d'octyle 10 %
- cire microcristalline 8 %
- cire de candelilla 5 %
- cire d'abeille 5 %
- tricaprylate/caprate de glycérol 4 %
- octyl méthoxy cinnamate 3 %
- ricinoléate de cétyle 3 %
- oxydes de fer 5 %
- pigments organiques sur laques 1,5 %
- nacres 6 %
- parfum 0,3%
- huile de ricin avec conservateur qsp 100 %

Cette composition est préparée comme dans l'exemple 1.

### Exemple 3 selon l'invention

### Composition cosmétique selon l'invention de maquillage de type poudre compacte

Cette composition est formulée à partir des ingrédients suivants :
- composition binaire de l'exemple 1 1,5 %
- nylon-12 6 %
- stéarate de calcium 2 %
- palmitate d'octyle 1 %
- octanoate de cétostéaryle 0,5 %
- mica traité silicone 15 %
- agents conservateurs tels que parahydroxybenzoate de méthyle, de propyle ou de butyle 0,25 %
- agents anti-oxydants tels que le gallate de propyle 0,2 %
- mica qsp 100 %

Cette composition est réalisée en mélangeant l'ensemble des composants dans un mélangeur adapté à la préparation de poudre, et en compactant dans un dispositif de compactage classique utilisé en cosmétique pour l'obtention d'une poudre compacte.

### Exemple 4 selon l'invention

### Composition cosmétique sous forme de fond de teint eau dans silicone

### I - Phase grasse'

- composition binaire de l'exemple 1 5 %
- laurylméthicone copolyol 3,5 %
- cyclométhicone 15 %
- octyldodécylstéaroylstéarate 5 %
- pigment traité silicone tel qu'oxydes de fer 8 %

### II - Phase aqueuse

- chlorure de sodium 2 %
- glycérine 3%
- agents conservateurs tels que parahydroxybenzoate de méthyle 0,15 %
- amidon modifié 4 %
- nylon-12 4 %
- eau qsp 100 %

On prépare cette composition de la manière suivante :

On mélange tout d'abord les composants de la phase grasse sous agitation jusqu'à obtention d'une homogénéité complète. Indépendamment, on mélange tous les composants de la phase aqueuse avec l'eau jusqu'à homogénéité complète.

On mélange ensuite la phase aqueuse et la phase grasse sous agitation vigoureuse et de façon progressive jusqu'à obtention d'une émulsion homogène.

On obtient ainsi une composition cosmétique de fond de teint comprenant des silicones et qui présente une bonne substantivité, une bonne tenue sur la peau, une bonne résistance à l'eau et une bonne résistance à la transpiration.

## Revendications

1. Utilisation d'une gomme de silicone de type diphényldiméthicone solubilisée dans une huile de silicone non volatile de type phényltriméthicone pour la fabrication d'une composition cosmétique ou pharmaceutique, notamment dermatologique, comprenant une phase grasse.

2. Utilisation selon la revendication 1, **caractérisée en ce que** la gomme de silicone de type diphényldiméthicone présente la formule (I) suivante : 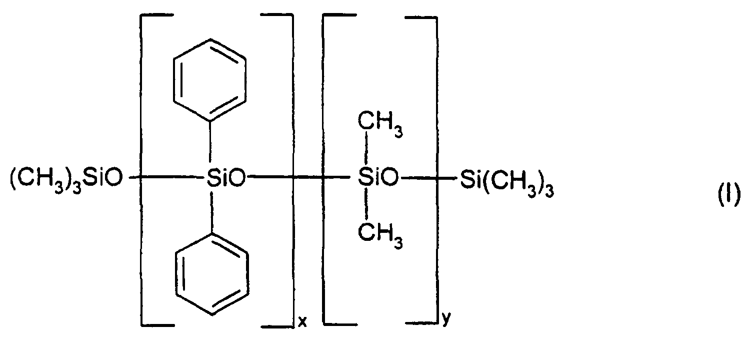 dans laquelle x représente le nombre de motifs de récurrence qui est généralement peu élevé, de préférence inférieur à environ 500, avantageusement compris entre environ 50 et environ 150, encore mieux compris entre environ 80 et environ 120, tandis que y représente le nombre de motifs de récurrence qui est généralement plus élevé, de préférence au moins égal à 1 000 et encore mieux compris entre environ 1 000 et environ 2 000.

3. Utilisation selon la revendication 1 ou 2, **caractérisée en ce que** l'huile silicone solubilisante non volatile de la gomme de silicone est une phényltriméthicone de formule (II) suivante : 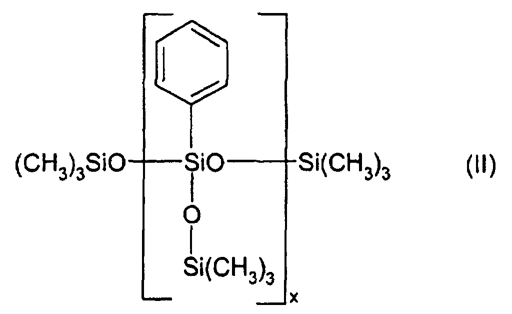 dans laquelle x représente le nombre de motifs de récurrence qui permet d'obtenir une viscosité comprise entre environ 0,1 m²/s et environ 0,4 m²/s (entre environ 10 et environ 40 centiStokes) à une température de 25°C; de préférence, x aura une valeur moyenne comprise entre 2 et 3.

4. Utilisation selon l'une des revendications précédentes, **caractérisée en ce que** la phase grasse est entièrement constituée du mélange binaire de la gomme de silicone précitée solubilisée dans l'huile de silicone non volatile de type phényltriméthicone.

5. Utilisation selon l'une des revendications 1 à 3, **caractérisée en ce que** le mélange binaire de la gomme de silicone et de l'huile de silicone solubilisante non volatile constitue de 0,1 à 100 %, mieux de 1 % à 75 %, encore mieux de 5 à 50 % de la phase grasse.

6. Utilisation selon l'une des revendications précédentes, **caractérisée en ce que** la phase grasse comprend au moins une autre huile silicone ou non silicone et éventuellement un corps gras qui peut être avantageusement d'origine végétale.

7. Utilisation selon la revendication 6, **caractérisée en ce que** l'huile non silicone précitée comprend au moins un ester de glycérol et d'acide gras sous forme de mono-, di- ou tri-glycéride ; un ester gras d'acide et d'alcool, l'acide et/ou l'alcool étant gras.

8. Utilisation selon l'une des revendications précédentes, **caractérisée en ce que** la composition cosmétique ou pharmaceutique, notamment dermatologique précitée comprend en outre des agents tensioactifs ou émulsionnants de type ionique ou non ionique, tels que des esters de sorbitan, des alcools gras polyoxyéthylénés, des esters de sucrose.

9. Utilisation selon l'une des revendications précédentes, **caractérisée en ce que** la composition cosmétique ou pharmaceutique, notamment dermatologique précitée peut également contenir au moins un additif cosmétiquement ou pharmaceutiquement, notamment dermatologiquement acceptable, tel qu'un agent épaississant, un ester d'acide gras en particulier des esters d'acide gras et de glycérol, d'autres huiles silicones volatiles ou non volatiles, des filtres solaires, des huiles végétales, des huiles synthétiques, des parfums, des agents conservateurs.

10. Utilisation selon l'une des revendications précédentes dans le domaine du soin de la peau, en particulier du visage ou du corps, ou pour le soin des cheveux.

11. Composition cosmétique, pharmaceutique, notamment dermatologique, **caractérisée en ce qu'**elle comprend une phase grasse comprenant une gomme de silicone de type diphényldiméthicone solubilisée dans une huile de silicone non volatile de type phényltriméthicone, en particulier telle que définie dans l'une quelconque des revendications 2 à 10.

12. Composition selon la revendication 11, **caractérisée en ce qu'**il s'agit d'une composition pour le soin de la peau qui comprend en outre dans la phase grasse un composé choisi parmi le groupe consistant de :
- un ester de glycérol, avantageusement un triglycéride liquide à la température ambiante, telle que le tricaprilate/caprate de glycérol, un triglycéride d'acide caprilique/caprique/succinique, en particulier à une concentration d'environ 1 à environ 10 % en poids,
- une huile de silicone de type phényltriméthicone ou diméthicone en particulier à une concentration comprise entre environ 1 et environ 10 % en poids, mieux de l'ordre de 2 à 5 % en poids,
- une huile végétale, avantageusement une huile de jojoba ou une huile de graine "d'herbe de la prairie" ("meadow foam seed oil"), en particulier à une concentration de 0,5 à 10 % en poids, mieux d'environ 1 à environ 5 % en poids,
- au moins un agent gélifiant, avantageusement un polysaccharide tel qu'une gomme de xanthane ou un polymère acrylique de type carbomer,
- ou un mélange gélifiant à base d'isoparaffine, de polyacrylamide et d'alcool laurique polyoxyéthyléné, en particulier à une concentration comprise entre environ 0,1 et environ 10 % en poids, mieux entre environ 0,1 et environ 5 % en poids,
- un agent émulsionnant/tensioactif de type non ionique tel que stéarate de sorbitan ou un stéarate de sorbitan polyoxyéthyléné ou un ester de sucrose tel que le stéarate de sucrose, en particulier à une concentration comprise entre environ 0,5 et environ 5 % en poids;

13. Composition selon la revendication 11, **caractérisée en ce qu'**elle est destinée à la protection solaire et comprend en outre les ingrédients suivants :
- au moins un filtre solaire chimique tel que le méthoxycinnamate d'octyle, le butylméthoxydibenzoylméthane, la benzophénone-3 et la benzophénone-4,
- au moins un filtre solaire physique tel que l'oxyde de titane micronisé en particulier à une concentration comprise entre 0,1 et 20 % en poids ; l'oxyde de zinc micronisé en particulier à une concentration comprise entre 0,1 et 20 % en poids, ou toutes particules hybrides réalisées à partir d'un mélange d'oxyde de titane ou de zinc avec un aut-e composant en particulier lorsque ces oxydes sont fixés à la surface d'une particule polymérique, par exemple des particules de polyéthylène ou de polypropylène comportant à leur surface de tels oxydes.

14. Composition selon la revendication 13, **caractérisée en ce qu'**elle comprend entre 0,1 et 10 % en poids de méthoxycinnamate d'octyle ; de 0,1 à 5 % en poids, en particulier de l'ordre de 2 à 3 % en poids, de butylméthoxydibenzoylméthane, de 0,1 à 5 % en poids de benzophénone-3 ou benzophénone-4
- au moins un filtre solaire physique tel que l'oxyde de titane micronisé en particulier à une concentration comprise entre 0,1 et 20 % en poids ; l'oxyde de zinc micronisé en particulier à une concentration comprise entre 0,1 et 20 % en poids, ou toutes particules hybrides réalisées à partir d'un mélange d'oxyde de titane ou de zinc avec un autre composant en particulier lorsque ces oxydes sont fixés à la surface d'une particule polymérique, par exemple des particules de polyéthylène ou de polypropylène comportant à leur surface de tels oxydes.

## Claims

1. Use of a silicone gum of the diphenyldimethicone type dissolved in a non-volatile silicone oil of the phenyltrimethicone type for preparing a cosmetic or pharmaceutical composition, notably dermatological composition, comprising a fatty phase.

2. Use according to claim 1, **characterised in that** the silicone gum of the diphenyldimethicone type has the following formula (I): 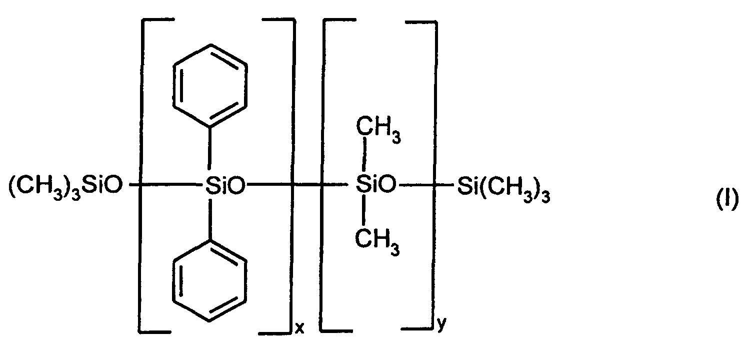 in which x represents the number of recurring units which is generally not very high, preferably less than about 500, advantageously between about 50 and about 150, even better between about 80 and about 120, while y represents the number of recurring units which is generally higher, preferably at least equal to 1,000 and even better between about 1,000 and about 2,000.

3. Use according to claim 1 or 2, **characterised in that** the non-volatile solubilising silicone oil of the silicone gum is a phenyltrimethicone of the following formula (II): 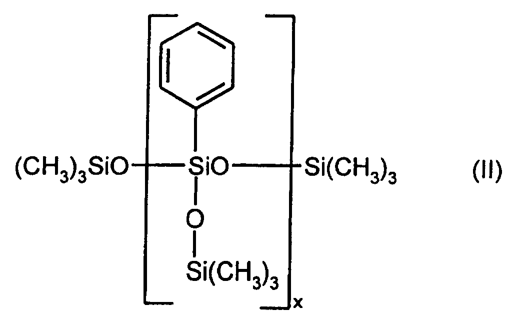 in which x represents the number of recurring units which enables a viscosity to be obtained between about 0.1 m²/s and about 0.4 m²/s (between about 10 and about 40 centiStokes) at a temperature of 25°C ; preferably, x will have an average value of between 2 and 3.

4. Use according to one of the preceding claims, **characterised in that** the fatty phase is constituted entirely of the binary mixture of the silicone gum mentioned above dissolved in the non-volatile silicone oil of the phenyltrimethicone type.

5. Use according to one of claims 1 to 3, **characterised in that** the binary mixture of the silicone gum and the solubilising non-volatile silicone oil constitutes 0.1 to 100 %, better 1 % to 75 %, even better 5 to 50 % of the fatty phase.

6. Use according to one of the preceding claims, **characterised in that** the fatty phase comprises at least one other silicone oil or non-silicone oil and optionally a fatty body which can advantageously be of plant origin.

7. Use according to claim 6, **characterised in that** the non-silicone oil mentioned above comprises at least one ester of glycerol and fatty acid in the form of a mono-, di- or tri-glyceride; a fatty ester of acid and alcohol, the acid and/or the alcohol being fatty.

8. Use according to one of the preceding claims, **characterised in that** the cosmetic or pharmaceutical composition, notably dermatological composition mentioned above further comprises surfactant or emulsifying agents of the ionic or non-ionic type, such as sorbitan esters, polyoxyethylenated fatty alcohols, sucrose esters.

9. Use according to one of the preceding claims, **characterised in that** the cosmetic or pharmaceutical composition, notably dermatological composition mentioned above can also contain at least one cosmetically or pharmaceutically acceptable additive, notably dermatologically acceptable additive, such as a thickening agent, a fatty acid ester particularly esters of fatty acid and glycerol, other volatile or non-volatile silicone oils, solar filters, plant oils, synthetic oils, perfumes, preservatives.

10. Use according to one of the preceding claims in the field of care of the skin, particularly of the face or of the body, or for hair care.

11. A cosmetic, pharmaceutical, notably dermatological composition, **characterised in that** it comprises a fatty phase comprising a silicone gum of the diphenyldimethicone type dissolved in a non-volatile silicone oil of the phenyltrimethicone type, particularly as defined in any one of claims 2 to 10.

12. The composition according to claim 11, **characterised in that** it is a composition for the care of the skin which further comprises in the fatty phase a compound selected from the group consisting of:
- an ester of glycerol, advantageously a triglyceride which is liquid at ambient temperature, such as glycerol tricaprylate/caprate, a caprylic/capric/succinic acid triglyceride, particularly at a concentration of about 1 to about 10 % by weight,
- a silicone oil of the phenyltrimethicone or dimethicone type particularly at a concentration between about 1 and about 10 % by weight, better of the order of 2 to 5 % by weight,
- a plant oil, advantageously a jojoba oil or meadow foam seed oil, particularly at a concentration of 0.5 to 10 % by weight, better of about 1 to about 5 % by weight,
- at least one gelling agent, advantageously a polysaccharide such as a xanthan gum or an acrylic polymer of the carbomer type,
- or a gelling mixture based on isoparaffin, polyacrylamide and polyoxyethylenated lauric alcohol, particularly at a concentration between about 0.1 and about 10 % by weight, better between about 0.1 and about 5 % by weight,
- an emulsifying/surfactant agent of the non-ionic type such as sorbitan stearate or a polyoxyethylenated sorbitan stearate or a sucrose ester such as sucrose stearate, particularly at a concentration between about 0.5 and about 5 % by weight.

13. The composition according to claim 11, **characterised in that** it is intended for solar protection and further comprises the following ingredients:
- at least one chemical solar filter such as octyl methoxycinnamate, butylmethoxydibenzoylmethane, benzophenone-3 and benzophenone-4,
- at least one physical solar filter such as micronised titanium oxide particularly at a concentration between 0.1 and 20 % by weight; micronised zinc oxide particularly at a concentration between 0.1 and 20 % by weight, or any hybrid particles made from a mixture of titanium oxide or zinc oxide with another component particularly when these oxides are fixed on the surface of a polymeric particle, for example particles of polyethylene or of polypropylene comprising such oxides on their surface.

14. The composition according to claim 13, **characterised in that** it comprises between 0.1 and 10 % by weight of octyl methoxycinnamate ; 0.1 to 5 % by weight, particularly of the order of 2 to 3 % by weight, of butylmethoxydibenzoylmethane, 0.1 to 5 % by weight of benzophenone-3 or benzophenone-4;
- at least one physical solar filter such as micronised titanium oxide particularly at a concentration between 0.1 and 20 % by weight; micronised zinc oxide particularly at a concentration between 0.1 and 20 % by weight, or any hybrid particles made from a mixture of titanium oxide or zinc oxide with another component particularly when these oxides are fixed on the surface of a polymeric particle, for example particles of polyethylene or of polypropylene comprising such oxides on their surface.

## Patentansprüche

1. Verwendung eines Silicongummis vom Diphenyldimethicon-Typ, gelöst in einem nichtflüchtigen Siliconöl vom Phenyltrimethicon-Typ, zur Herstellung einer kosmetischen oder pharmazeutischen, insbesondere dermatologischen Zusammensetzung mit einer Fettphase.

2. Verwendung nach Anspruch 1, **dadurch gekennzeichnet, dass** der Silicongummi vom Diphenyldimethicon-Typ die folgende Formel (I) hat: 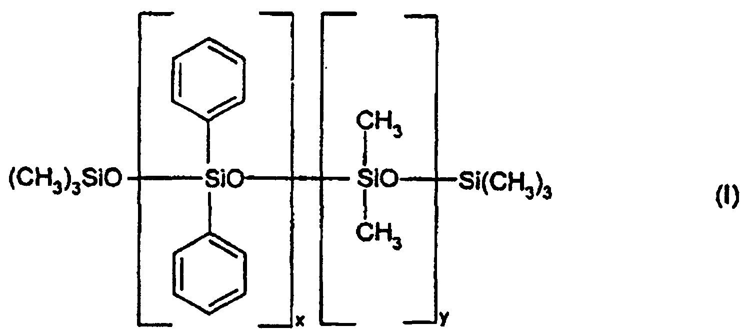 worin x die Anzahl an wiederkehrenden Komponenten darstellt, die im Allgemeinen nicht sehr hoch, vorzugsweise kleiner als etwa 500 ist und vorteilhaft zwischen etwa 50 und etwa 150, noch besser zwischen etwa 80 und etwa 120 liegt, während y die Anzahl an wiederkehrenden Komponenten darstellt, die im Allgemeinen höher, vorzugsweise mindestens gleich 1000 ist und noch besser zwischen etwa 1000 und etwa 2000 liegt.

3. Verwendung nach Anspruch 1 oder 2, **dadurch gekennzeichnet, dass** das nichtflüchtige Siliconöl zum Lösen des Silicongummis ein Phenyltrimethicon der folgenden Formel (II) ist: 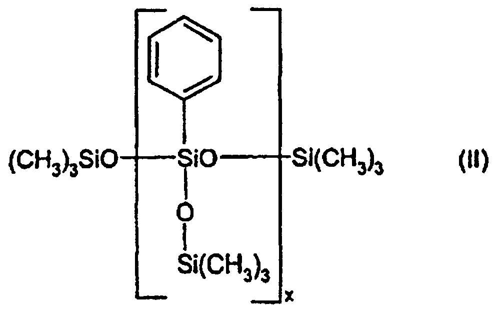 worin x die Anzahl an wiederkehrenden Komponenten darstellt, die eine Viskosität zwischen etwa 0,1 m²/s und etwa 0,4 m²/s (zwischen etwa 10 und etwa 40 Zentistoke) bei einer Temperatur von 25 °C erzielen lassen und x vorzugsweise einen Mittelwert zwischen 2 und 3 aufweist.

4. Verwendung nach einem der vorhergehenden Ansprüche, **dadurch gekennzeichnet, dass** die Fettphase zur Gänze aus der binären Mischung des im nichtflüchtigen Siliconöl vom Phenyltrimethicon-Typ gelösten Silicongummis gebildet ist.

5. Verwendung nach einem der Ansprüche 1 bis 3, **dadurch gekennzeichnet, dass** die binäre Mischung aus Silicongummi und nichtflüchtigem lösendem Siliconöl 0,1 bis 100 %, besser 1 bis 75 %, noch besser 5 bis 50 % der Fettphase ausmacht.

6. Verwendung nach einem der vorhergehenden Ansprüche, **dadurch gekennzeichnet, dass** die Fettphase mindestens ein weiteres Öl, u.zw. entweder ein Siliconöl oder ein siliconfreies Öl, und gegebenenfalls einen Fettkörper umfasst, der vorteilhaft pflanzlichen Ursprungs sein kann.

7. Verwendung nach Anspruch 6, **dadurch gekennzeichnet, dass** das siliconfreie Öl mindestens einen Glycerin- und Fettsäureester in Form von Mono-, Di- oder Triglycerid; einen Säure- und Alkoholfettester umfasst, wobei die Säure und/oder der Alkohol eine Fettsäure bzw. ein Fettalkohol ist.

8. Verwendung nach einem der vorhergehenden Ansprüche, **dadurch gekennzeichnet, dass** die kosmetische oder pharmazeutische, insbesondere dermatologische Zusammensetzung weiters ionische oder nichtionische grenzflächenaktive Mittel oder Emulgiermittel wie Sorbitanester, Polyoxyethylen-Fettalkohole, Saccharoseester umfasst.

9. Verwendung nach einem der vorhergehenden Ansprüche, **dadurch gekennzeichnet, dass** die kosmetische oder pharmazeutische, insbesondere dermatologische Zusammensetzung auch mindestens einen kosmetisch oder pharmazeutisch, insbesondere dermatologisch annehmbaren Zusatzstoff wie ein Verdickungsmittel, einen Fettsäureester, insbesondere Fettsäure- und Glycerinester, weitere gegebenenfalls flüchtige Siliconöle, Sonnenfilter, pflanzliche Öle, synthetische Öle, Parfums, Konservierungsmittel enthalten kann.

10. Verwendung nach einem der vorhergehenden Ansprüche auf dem Gebiet der Hautpflege, insbesondere Gesichts- oder Körperpflege, oder zur Pflege der Haare.

11. Kosmetische, pharmazeutische, insbesondere dermatologische Zusammensetzung, **dadurch gekennzeichnet, dass** sie eine Fettphase mit einem Silicongummi vom Diphenyldimethicon-Typ, gelöst in einem nichtflüchtigen Siliconöl vom Phenyltrimethicon Typ, insbesondere wie in einem der Ansprüche 2 bis 10 definiert, enthält.

12. Zusammensetzung nach Anspruch 11, **dadurch gekennzeichnet, dass** es sich um eine Zusammensetzung zur Hautpflege handelt, die in der Fettphase weiters eine Verbindung enthält, die ausgewählt ist aus der Gruppe bestehend aus:
- einem Glycerinester, vorteilhaft einem bei Umgebungstemperatur flüssigen Triglycerid wie Tricaprilat/Glycerincaprat, einem Triglycerid von Capryl-/Caprin-/Bernsteinsäure, insbesondere in einer Konzentration von etwa 1 bis etwa 10 Gew.-%,
- einem Siliconöl vom Phenyltrimethicon- oder Dimethicon-Typ, insbesondere in einer Konzentration zwischen etwa 1 und etwa 10 Gew.-%, besser im Bereich von 2 bis 5 Gew.-%,
- einem pflanzlichen Öl, vorteilhaft Jojobaöl oder "Wiesenschaumkraut"-Saatöl ("meadow foam seed oil"), insbesondere in einer Konzentration von 0,5 bis 10 Gew.-%, besser etwa 1 bis etwa 5 Gew.-%,
- mindestens einem Geliermittel, vorteilhaft einem Polysaccharid wie Xanthangummi oder einem Acrylpolymer vom Carbomer-Typ,
- oder einer gelbildenden Mischung auf Isoparaffin-, Polyacrylamid- und Polyoxyethylenlaurylalkoholbasis, insbesondere in einer Konzentration zwischen etwa 0,1 und etwa 10 Gew.%, besser zwischen etwa 0,1 und etwa 5 Gew.-%,
- einem Emulgiermittel/grenzflächenaktiven Mittel vom nichtionischen Typ wie Sorbitanstearat oder Polyoxyethylensorbitanstearat oder einem Saccharoseester wie Saccharosestearat, insbesondere in einer Konzentration zwischen etwa 0,5 und etwa 5 Gew.-%.

13. Zusammensetzung nach Anspruch 11, **dadurch gekennzeichnet, dass** sie als Sonnenschutz gedacht ist und weiters folgende Inhaltsstoffe umfasst:
- mindestens einen chemischen Sonnenfilter wie Octylmethoxycinnamat, Butylmethoxydibenzoylmethan, 3-Benzophenon und 4-Benzophenon,
- mindestens einen physikalischen Sonnenfilter wie mikronisiertes Titanoxid, insbesondere in einer Konzentration zwischen 0,1 und 20 Gew.-%, mikronisiertes Zinkoxid, insbesondere in einer Konzentration zwischen 0,1 und 20 Gew.-%, oder jede anderen Hybridteilchen, die aus einer Mischung von Titan- oder Zinkoxid mit einem anderen Bestandteil hergestellt sind, insbesondere wenn diese Oxide an der Oberfläche eines Polymerteilchens fixiert sind, beispielsweise Polyethylen- oder Polypropylenteilchen, die an ihrer Oberfläche derartige Oxide aufweisen.

14. Zusammensetzung nach Anspruch 13, **dadurch gekennzeichnet, dass** sie zwischen 0,1 und 10 Gew.-% Octylmethoxycinnamat, 0,1 bis 5 Gew.-%, insbesondere 2 bis 3 Gew.-% Butylmethoxydibenzoylmethan, 0,1 bis 5 Gew.-% 3-Benzophenon- oder 4-Benzophenon,
- mindestens einen physikalischen Sonnenfilter wie mikronisierte Titanoxid, insbesondere in einer Konzentration zwischen 0,1 und 20 Gew.-%, mikronisiertes Zinkoxid, insbesondere in einer Konzentration zwischen 0,1 und 20 Gew.-%, oder jede anderen Hybridteilchen umfasst, die aus einer Mischung von Titan- oder Zinkoxid mit einem anderen Bestandteil hergestellt sind, insbesondere wenn diese Oxide an der Oberfläche eines Polymerteilchens fixiert sind, beispielsweise Polyethylen- oder Polypropylenteilchen, die an ihrer Oberfläche derartige Oxide aufweisen.
